# EUROPEAN PATENT SPECIFICATION

(11) **EP 4 103 254 B1**
(45) Date of publication and mention of the grant of the patent: **09.07.2025**
(21) Application number: 21704551.7
(22) Date of filing: 12.02.2021
(51) Int. Cl.: A61M 16/00

(54) **SYSTEM AND METHOD FOR CONTROLLING A TRACHEOBRONCHIAL-AIR STIMULATION DEVICE**
SYSTEM UND VERFAHREN ZUR STEUERUNG EINER TRACHEOBRONCHIALLUFTSTIMULATIONSVORRICHTUNG
SYSTÈME ET PROCÉDÉ POUR COMMANDER UN DISPOSITIF DE STIMULATION DE L'AIR TRACHÉOBRONCHIQUE

(30) Priority: 13.02.2020 EP 20305136
(43) Date of publication of application: 21.12.2022
(73) Proprietor: Physio-Assist, 13593 Aix-en-Provence (FR)
(72) Inventor: MITHALAL, Adrien, 13593 Aix-en-Provence Cedex 3 (FR)
(74) Representative: Icosa
(86) International application number: PCT/EP2021/053400
(87) International publication number: WO 2021/160775

(56) References cited:
- WO-A1-2017/009299
- WO-A2-2010/058308
- US-A1- 2016 287 823
- US-B1- 6 176 235

## Description

### FIELD OF INVENTION

The present disclosure pertains to the field of tracheobronchial-air stimulation devices, in particular for subjects with respiratory disorders. More particularly, the disclosure relates to an automatic control system for a tracheobronchial-air stimulation device intended to be connected to a subject, as well as a method for controlling a tracheobronchial-air stimulation device. The technology finds a particularly advantageous, although non-limiting, application for subjects who are not assisted on a regular basis by a competent operator or a physician.

### BACKGROUND OF INVENTION

For a healthy individual, lungs are covered by a "film" called mucus, which is a few millimetres thick and has a very fluid normal consistency. The function of this mucus is to protect lung cells by isolating them from direct contact with the air inhaled by the lungs. The renewal of this mucus is ensured by mobile cilia located on the surface of the bronchi. These cilia, whose movements are described as vibratile, beat towards the proximal part of the airways, thus eliminating inhaled particles trapped in the freezing phase of the mucus, which slide on the cilia layer. Such a kinematics, known as mucociliary clearance (i.e. the ability of a tissue, organ or organism to remove a given substance from a fluid) is carried out at a speed of about 5 mm/min in the trachea, ensuring the renewal of the mucus layer every 20 minutes approximately. This mucociliary clearance allows the mucus to be eliminated towards the pharynx where it is coughed up or swallowed.

Different pathologies result in an obstructive respiratory disorder, or obstructive pulmonary syndrome, which is characterized by an accumulation of mucus. Consequences of such an accumulation of mucus are, notably, a limitation of flow rates in the bronchial tree and an increase in aerial resistance, which contribute to the emergence of potentially severe infections. Obstructive respiratory disorders may result from a chronic condition corresponding to bronchiectasis (also known as bronchial dilation, or BD), most often acquired as a result of bronchial, lung or pleural disease.

Bronchiectasis, which may be localized or diffuse, is characterized by a dilation of the small and medium-sized bronchi and is often associated with abundant mucopurulent expectoration, which reflects an over-infection. Possible causes of bronchiectasis are multiple and include cystic fibrosis, COPD (e.g. pulmonary emphysema or chronic bronchitis), including severe early childhood infections (e.g. asthma or bronchiolitis), ciliary dyskinesia, bronchial stenosis, consequences of pulmonary tuberculosis, or congenital or acquired Ig deficiencies (A, G, or M).

In order to facilitate the circulation and evacuation of mucus, different solutions have been used so far. For example, use of mucolytic agents as well as mucus regulator is well known. Yet, such solutions are only partial and make it possible, at most, to avoid over-infection. Therefore, they are often complemented with physiotherapy sessions which, however, may be traumatic for a subject, and may also have limited efficiency when the mucus is too viscous or elastic.

There also exist other solutions using stimulation devices configured to stimulate the tracheobronchial air of a subject. Such stimulation devices include a pressure unit configured to generate pressure pulses, either positive or negative (negative pressure pulses being also known as "underpressures"), as well as connection means configured to connect the pressure unit to the respiratory system of the subject, such as a tube preferably associated with a tip to be inserted in the mouth of the subject. Within the frame of the invention, the expression "respiratory system of the subject" refers to airways of the subject, including ducts such as the pharynx, larynx and bronchi, as well as the lungs themselves.

Operation of such stimulation devices involves the delivery of positive pressure pulses during inhalation phases, and the delivery of negative pressure pulses during exhalation phases. The objective is to stimulate a cough of the subject during the exhalation phases, so as to cause the mucus to separate from the bronchial walls. However, such a process is difficult to implement, first because coughing may cause physical discomfort for the subject, especially when the mucus is not sufficiently elastic, and also because the amplitude of the pulses can lead to lung collapse, which may have serious consequences on the health of the subject.

More recently, it has been proposed to adjust the use of such stimulation devices by applying underpressures during relaxed exhalation phases in alternance with phases of return to ambient pressure, *i.e.,* the subject inhales after removing said connection means from his/her mouth, as proposed in the international patent application WO 2017/009299. Within the scope of the technology, "relaxed exhalation" refers to an exhalation supported by the stimulation device via said negative pressure pulses. Such a process advantageously makes it possible to avoid cough and lung collapse. However, the results obtained with regard to the efficiency of mucus removal are not yet optimal. Indeed, while cough is avoided, the operation of the stimulation device implies, during each relaxed exhalation, a continuous increase in the underpressure applied to the respiratory system of the subject. This increase is likely to cause discomfort to the patient, thus preventing optimal emptying of the lungs. As a result, the distal airways cannot be reached, and the associated mucus cannot be removed.

### SUMMARY

The invention is defined by the appended claims.

The purpose of the present technology is to overcome all or some of the limitations of the prior art solutions, by providing a system and a method making it possible to maximize an exhalation time of a subject over a relaxed exhalation performed by means of a tracheobronchial-air stimulation device, so that the lungs of the subject are emptied as much as possible to improve efficiency of the stimulation (*i.e.* applying the larger underpressure acceptable), while considering subject's tolerance to underpressures (*i.e.* avoiding cough). Stimulation is improved with a real-time control of underpressure.

To this end, according to a first aspect, a subject of the invention is an automatic control system for a tracheobronchial-air stimulation device intended to be connected to a subject, the stimulation device comprising a pressure unit configured to generate a set of negative pressure pulses during a relaxed exhalation of the subject, each pulse corresponding to a power of the pressure unit, and a connection assembly configured to connect the pressure unit to the respiratory system of the subject, wherein the control system comprises:
- a pressure sensor configured to measure, after each pulse generated by the pressure unit, an inner underpressure applied to the respiratory system of the subject corresponding to said pulse,
- a calculation module configured to compare in real-time the absolute value of the measured inner underpressure with a predetermined threshold and, based on the comparison result, determine an updated power value to be applied to the pressure unit so that the absolute value of a subsequent inner underpressure corresponding to the updated power value is below the absolute value of the threshold,
- a control module configured to apply the updated power value to the pressure unit before generation of the next pulse.

By "respiratory system of the subject", it is referred to the set of airways of the subject, including ducts such as the pharynx, larynx and bronchi, as well as the lungs themselves.

By "relaxed exhalation", it is referred to the fact that the extraction of air from the lungs of the subject is carried out by means of said pulses alone, i.e. without the subject exhaling by his/her own will. Furthermore, by "power", it is referred to a quantity representative of the volume of air extracted for each negative pressure pulse. Thus, if such power increases/decreases, it corresponds to a situation where the extracted air volume increases/decreases for underpressures due to the increase/decrease in power. Here, if underpressure increases, it means that absolute value of underpressure increases; in other words, a lower pressure is imposed to the subject when underpressure increases.

By "inner underpressure", it is referred to the pressure actually felt by the subject, *i.e*. the pressure in the mouth, at the entrance of airways, of the subject. Typically, the value of inner underpressure is in the range from -10 millibars to -100 millibars in operation of the device. Inner underpressure may be different from the expected negative pressure pulse produced by the pressure unit with a control power value. Indeed, a given power value of the pressure unit generates a theoretical negative pressure - typically in the range from -300 millibars for full power value to 0 millibars with minimum power value - but actual negative pressure is influenced by various features: tubing, stage of exhalation and actual subject anatomy among others. Inner underpressure is the relevant parameter for the automatic control system.

By "real-time", it is meant that the comparison of the absolute value of the measured inner underpressure (during current negative pressure pulse) with the threshold is quick enough to modify the order sent to the pressure unit for next negative pressure pulse. For negative pressure frequency of 12 Hz, comparison should be done in less than 80 ms by the calculation module. For negative pressure frequency of 6 Hz, comparison should be done in less than 160 ms by the calculation module. Inner underpressure may be measured at a sampling rate of 100 Hz, for comparison and control of pressure unit.

Thus, the system is based first on the measurement in real time of said inner underpressure, yielding the actual underpressure felt by the subject, which may be different from the negative pressure imposed by the pressure unit. The calculation module uses this measured inner underpressure to compare it to a threshold, and then determines a power value to ensure that the next negative pressure pulse remains below the threshold.

In this way, if the inner underpressure associated with a pulse exceeds the threshold, this excess does not continue at least for the next pulsation. In other words, this prevents the inner underpressure from being maintained for too long above the threshold, so that the relaxed exhalation is not stopped prematurely by the subject, for example because he/she feels the need to cough. In the end, the relaxed exhalation is carried out for a sufficiently long period of time so that the subject completely empties the air contained in his/her lungs, which facilitates the drainage of mucus during a relaxed exhalation.

Furthermore, as long as the threshold is not exceeded, such a system prevents the inner underpressure from being maintained for too long at a too low level. In other words, it prevents that too little volumes air are removed from the lungs of the subject, thus increasing the efficiency of mucus drainage during a relaxed exhalation.

Finally, with this automatic control system, underpressure applied to the subject never exceeds acceptable value below the threshold for which the subject would start coughing, during a whole relaxed exhalation.

In particular embodiments, the system may additionally include one or more of the following features, either taken in isolation or according to any technically possible combinations.

According to one embodiment, the system further comprises an acquisition module configured to acquire, during a relaxed exhalation of the subject and after each pulse generated by the pressure unit, a value of the measured inner underpressure associated with said pulse.

According to one embodiment, the calculation module is configured to determine if the absolute value of the measured inner underpressure exceeds the absolute value of the threshold, and, in case of exceeding, determine a decreased power value to be applied to the pressure unit. Thus, the comparison makes it possible to detect an exceeding of the threshold, which then ensures that the calculation module generates an appropriate power value in order to optimize the operation of the stimulation device.

According to one embodiment, the calculation module is configured to determine the decreased power value as a function of a difference between the absolute value of the measured inner underpressure and the absolute value of the threshold, so that the absolute value of a subsequent inner underpressure corresponding to said decreased power value is decreased by a value of between 10 mbar et 60 mbar compared to the latter measured inner underpressure. The choice of such a range advantageously reduces the inner underpressure sufficiently, so that the subject does not feel any respiratory discomfort and can therefore continue to use the stimulation device. In other words, the subject does not feel the need to remove the tip from the mouth, so that the lungs continue to be emptied, which enhances the extraction of air from the lungs. In addition, targeting such particular range allows the operation of the stimulation device to be finely controlled.

According to one embodiment, the decreased power value is determined among a set of predetermined power values. Such a configuration makes it possible to simplify the electronic architecture of the system since the decreased power values can be pre-programmed.

According to one embodiment, said predetermined values are substantially equal to multiples of one-tenth of the maximum power of the pressure unit. The choice of such values makes it possible to obtain a good efficiency while keeping a simplified electronic architecture.

According to one embodiment, when several decreased power values are determined by the calculation module during the relaxed exhalation of the subject, said values form a constant sequence over time. Such a configuration ensures that the subject is not rushed during a relaxed exhalation, since he/she is always confronted with a same power decrease. As a result, the subject has a smoother interaction with the stimulation device, which improves the comfort and resulting efficiency.

According to one embodiment, the calculation module is configured to determine whether the absolute value of the measured inner underpressure exceeds the absolute value of the threshold, and, in case of no exceeding, determine an increased power value to be applied to the pressure unit. Thus, the comparison makes it possible to detect, in this case, that the threshold is not reached, which then ensures that the control module generates an appropriate command in order to optimize the operation of the stimulation device.

According to one embodiment, the calculation module is configured to determine the increased power value as a function of a difference between the absolute value of the measured inner underpressure and the absolute value of the threshold, so that the subsequent inner underpressure corresponding to said increased power value is increased by a value of between 10 mbar et 60 mbar compared to the latter measured inner underpressure. The choice of such a range advantageously prevents the stimulation device from operating at a too low power rate, which would lead to not enough air being extracted from the lungs with each pulse. In other words, proceeding in this way increases the performance and efficiency of the device by extracting the optimum volume of air during each relaxed exhalation, until the threshold has been reached.

According to one embodiment, the pressure unit is configured in such a way that, for a plurality of relaxed exhalations of the subject, a first set of consecutive exhalations is associated with pulses generated by the pressure unit at a first frequency, whereas a second set of consecutive exhalations is associated with pulses generated by the pressure unit at a second frequency distinct from the first frequency.

According to one embodiment, the first frequency is equal to 12 Hz and the second frequency is equal to 6 Hz. These frequencies respectively enhance the liquefaction power and the drainage power of the mucus.

According to one embodiment, the threshold is a value corresponding to a balance between performance (*i.e.* optimum extraction of air from the lungs) and comfort limit determined during use of the stimulation device by the subject. Such a configuration makes it possible to better respect the tolerance of the subject with regard to the operation of the stimulation device, thus facilitating prolonged use of the latter and therefore optimal efficiency.

According to one embodiment, the threshold is a predefined value determined during a test phase of the stimulation device. Such a configuration makes it possible to increase the autonomy of the subject with regard to the use of the stimulation device, since once the test phase is over, the presence of a physician is no longer required.

According to a second aspect, a subject of the invention is an assembly comprising:
- a tracheobronchial-air stimulation device intended to be connected to a subject, the stimulation device including a pressure unit configured to generate, during a relaxed exhalation of the subject, a set of negative pressure pulses, each pulse corresponding to a power of the pressure unit, and a connection assembly configured to connect the pressure unit to the respiratory system of the subject, and
- an automatic control system as described above.

According to a third aspect of the disclosed technology (not claimed), there is described a method for automatically controlling a tracheobronchial-air stimulation device as a function of measurements of an inner underpressure, the stimulation device comprising a pressure unit configured to generate a set of negative pressure pulses during a relaxed exhalation of a subject, each pulse corresponding to a power of the pressure unit, and a connection assembly configured to connect the pressure unit to the respiratory system of the subject, wherein the method comprises steps of:
- measuring, after each pulse generated by the pressure unit, an inner underpressure applied to the respiratory system of the subject corresponding to said pulse,
- comparing in real-time the absolute value of the measured inner underpressure with a predetermined threshold and, based on the comparison result, determining an updated power value to be applied to the pressure unit so that the absolute value of a subsequent inner underpressure corresponding to the updated power value is below the absolute value of the threshold,
- applying the updated power value to the pressure unit before generation of the next pulse.

According to a fourth aspect, a subject of the disclosure is a computer program comprising instructions for the implementation of at least the calculation steps of a method according to the technology when the program is executed by a computer.

According to a fifth aspect, a subject of the disclosure is a non-transitory computer readable medium comprising instructions for the implementation of at least the calculation steps of a method according to the invention when the instructions are executed by a computer.

### BRIEF DESCRIPTION OF THE DRAWINGS

Features and advantages of the invention will become apparent from the following description of embodiments of a system and a method (not claimed) for controlling a tracheobronchial air stimulation device, this description being given merely by way of example and with reference to the appended drawings in which:
**Figure 1** is a schematic representation of an assembly comprising a tracheobronchial-air stimulation device and an automatic control system according to the technology.
**Figure 2** is a graph illustrating over time two pulses emitted successively during a relaxed exhalation.
**Figure 3** is a graph showing the evolution over time of the inner underpressure during a relaxed exhalation, in a case where the tracheobronchial-air stimulation device is controlled according to the prior art.
**Figure 4** is a diagram showing the main steps of a method for controlling a tracheobronchial-air stimulation device.
**Figure 5** is a graph showing the evolution over time of the inner underpressure during a relaxed exhalation, for a system and a method according to a first embodiment of the disclosure corresponding to an automatic control of the tracheobronchial-air stimulation device in which the threshold is exceeded twice.
**Figure 6** is a graph showing the evolution over time of the inner underpressure during a relaxed exhalation, for a system and a method according to a second embodiment of the disclosure corresponding to an automatic control of the tracheobronchial-air stimulation device in which the amplitude of the inner underpressure oscillates around the threshold.
**Figure 7** is a graph illustrating a variant of the embodiment of Figure 5, where only a part of a relaxed exhalation is represented, and where the control module generates a power increase command during said part of the relaxed exhalation.
**Figure 8** is a graph illustrating the power value W of the pressure unit (as a percentage of full power of pressure unit) versus time T (in seconds) during a relaxed exhalation with constant inner underpressure applied to the subject. Some plateaus are identified as W1, W2 and W3.
**Figure 9** is a graph illustrating showing the experimental measurement of the evolution over time (in seconds) of the inner underpressure during a relaxed exhalation, for a system and a method of prior art, without automatic control system.
**Figure 10** is a graph showing in the upper part the experimental measurement of the evolution over time (in seconds) of the inner underpressure during a relaxed exhalation, for a system and a method according to an embodiment of the disclosure corresponding to an automatic control of the tracheobronchial-air stimulation device in which the amplitude of the inner underpressure is kept constant (here -40 mBar - dotted line). The lower part of the figure is showing the power value reported on the same time axis.

Arrows show two occurrences of inner underpressure exceeding the threshold, immediately followed by a decrease in power value applied to the pressure unit.

In the figures, references that are identical from one figure to another denote identical or analogous elements. For reasons of clarity, the elements represented are not to scale, unless stated otherwise. Moreover, the figures are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

### DETAILED DESCRIPTION

This technology is part of the field of medical assistance to a subject suffering from respiratory disorders. The following description refers more specifically, but in a non-limited way, to the case of a subject suffering from bronchiectasis. In other words, the subject faces health problems related to the difficulty of extracting mucus accumulated in the bronchi from his/her lungs. Of course, a subject suffering from another pathology may also be considered if the latter requires assistance to relieve a respiratory problem.

In particular, the invention is also applicable in the case of a subject affected by a benign pathology, such as a cold, bronchitis, etc.

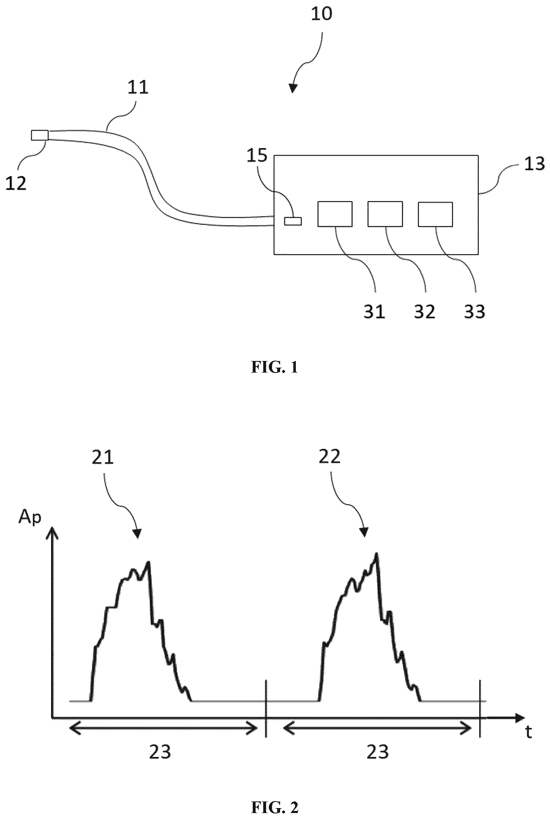

Figure 1 schematically illustrates an assembly comprising a tracheobronchial air stimulation device 10.

In a conventional way, the stimulation device 10 includes a pressure unit 13 and a connection assembly for connecting said pressure unit 13 to the respiratory system of a subject. Such connection assembly allows the circulation of air flows between the respiratory system of the subject and the other elements of the device 10 used to perform the stimulations, these elements being described in more detail below.

According to a non-limiting example, and as shown in Figure 1, the connection assembly includes a tube 11, for example made of plastic, so as to remain flexible and thus facilitate the positioning of the subject with respect to the device 10. The tube 11 comprises two ends, one of these being equipped with a tip 12, as part of the connection assembly, to be inserted into the mouth of the subject. The other end is connected to the pressure unit 13.

The pressure unit 13 is configured to generate, during a relaxed exhalation of the subject, a set of negative pressure pulses, each pulse being associated with a power of said pressure unit 13. The negative pressure pulses correspond to pulses during which a underpressure is created in the respiratory system of the subject so as to extract the air in his/her lungs, this air itself being the consequence of an inhalation that was previously carried out before the tip 12 of the tube was placed in mouth. Otherwise said, a negative pressure pulse corresponds to a pressure lower than a reference pressure, here considered, in a non-limiting example, to be the atmospheric pressure. In particular, it is noted that in the figures the atmospheric pressure corresponds to the "0" value on the P-axis.

It is noted that the power is associated with the pressure unit 13, but it also corresponds to the power of the stimulation device 10 itself. Thus, the expressions "power of the pressure unit" and "power of the stimulation device" have the same meaning in this description.

Typically, the stimulation device 10 is used by the subject to perform a plurality of relaxed exhalations during a medical assistance session, in order to dislodge as much mucus as possible and thus optimize the efficiency of the session. Such a session thus comprises a succession of relaxed exhalations, two successive relaxed exhalations being separated by an inhalation phase during which the tip 12 is removed from the mouth of the subject. For example, a session comprises ten relaxed exhalations, it being understood that a different number of relaxed exhalations can be considered.

According to a particular embodiment, the pressure unit 13 includes a vacuum pump and an electro-valve. The person skilled in the art is well aware of their respective operations, which are therefore not described further here. Furthermore, the pressure unit 13 may be configured differently, for example by means of a valve of a different type. Here again, the person skilled in the art is able to design alternatives.

In addition, the pressure unit 13 comprises, for example, one or more processors and storage means (magnetic hard disk, electronic memory, optical disk, etc.) in which data and a computer program product are stored, in the form of a set of program code instructions to be executed to implement all or part of the said pulse generation. Alternatively, or in addition, the generation unit 13 comprises one or more programmable logic circuits (FPGA, PLD, etc.), and/or one or more specialized integrated circuits (ASIC), and/or a set of discrete electronic components, etc. suitable for implementing all or part of the pulse generation according to predetermined features.

In other words, the pressure unit 13, in addition to means such as a vacuum pump and an electro-valve, comprises a set of means configured by software (specific computer program product) and/or hardware (FPGA, PLD, ASIC, etc.) in order to generate pulses according to predetermined features as described below.

Figure 2 schematically illustrates two pulses 21, 22 emitted successively during a relaxed exhalation of the subject, and corresponds to a graph representing time on the abscissa axis, and the underpressure (in millibars, typically the peak value of underpressure is in the range from -10 millibars to -80 millibars, preferably from -15 millibars to -40 millibars, more preferably from -20 millibars to -40 millibars, even more preferably from -30 millibars to -40 millibars), associated to each pulse, on the ordinate axis. It is noted that the representation in Figure 2 is given for illustrative purposes only, *i.e.* inter alia, the number of pulses during a relaxed exhalation is not limited to two.

As shown in Figure 2, each pulse is emitted during a time window 23 of a predetermined duration, the duration of a pulse being less than the duration of the associated time window. In this way, a cyclic ratio equal to the ratio between the pulse duration and the window duration is defined for each time window.

Preferably, and as illustrated in Figure 2, the time windows 23 associated respectively with the pulses emitted during a relaxed exhalation are all of the same duration. Proceeding in this way makes it possible to simplify the electronics for implementation of the invention, and therefore ultimately its cost, without compromising an efficient dislodging of the mucus.

According to a particular embodiment, the pressure unit 13 is configured to generate pulses at least at a predetermined frequency and also at least at a predetermined cyclic ratio. For example, and as shown in Figure 2, the emission frequency of both pulses is 12 Hz (hence the duration of the frequency window associated with each pulse is equal to 1/12th of a second), each pulse having a cyclic ratio of 0.3. Such respective values for the frequency and the cyclic ratio result in a short and strong underpressure so that a stronger liquefying power is obtained by thixotropy. Alternatively, the emission frequency of the two pulses is 6 Hz, each pulse being associated with a cyclic ratio equal to 0.6. Such respective values for frequency and cyclic ratio result in a lower and longer underpressure than if the frequency and cyclic ratio are equal to 12 Hz and 0.3 respectively. The liquefying power obtained is therefore less important, but this makes it possible to increase the drainage power in return by transmitting kinetic energy between the air and the mucus over a longer period of time.

According to yet another alternative, when a plurality of relaxed exhalations is carried out by the subject, part of said exhalations are associated with pulses generated at a first frequency f₁, whereas the other exhalations are associated with pulses generated at a second frequency f₂ distinct from the first frequency f₁, said exhalations associated with a given frequency being consecutive. By way of non-limiting example, the first frequency f₁ and the second frequency f₂ are respectively equal to 12 Hz and 6 Hz. In an even more specific example, the 12 Hz and 6 Hz frequencies are associated with cyclic ratios of 0.3 and 0.4 respectively. This makes it possible, during a full session of medical assistance, not only to dislodge the mucus but also to drain it. However, it is also possible to have different frequencies f₁ and f₂ as well as different cyclic ratios.

The choice of a particular emission frequency constitutes only a variant for implementing the invention. Also, and more generally, it will be clear for the person skilled in the art that a pulse emission can be considered that is not associated with any predetermined frequency. In the same way, the choice of a cyclic ratio equal to 0.3 or 0.6 is only a variant of the implementation of the invention. More generally, the cyclic ratio may be higher than 0.3.

The stimulation device 10 is controlled by a system configured for this purpose, and described in more detail below. By controlling the stimulation device 10, it is referred to the fact of modulating the power of this device 10 in such a way as to vary the power associated with each pulse, and thus to control the variation in the volume of air extracted from the lungs of the subject.

As illustrated in Figure 1, the system comprises a pressure sensor 15 configured to measure, at least after each pulse generated by the pressure unit 13, an inner underpressure P applied to the respiratory system of the subject. It must be understood that the inner underpressure P also corresponds to the air pressure at the other end of the tube 11, i.e. the end of the tube 11 attached to the pressure unit 13. Such a property is the result of the closure of the air circulation circuit between the respiratory system of the subject on the one hand, and the stimulation device 10 on the other hand.

Such a pressure sensor 15 is of a design known per se. In a general manner, the properties of pressure sensors are well known to the person skilled in the art who will be able to choose a pressure sensor 15 from a catalogue of products offered by specialized manufacturers. By way of a non-limiting example, the pressure sensor 15 is a calibrated pressure piezoelectric sensor with a measure range including values between +20 millibars to -300 millibars.

In the present embodiment, and as illustrated in Figure 1, the pressure sensor 15 is fixedly positioned inside the pressure unit 13, near the tube that will be inserted into the mouth of the subject. However, it is also possible, according to other examples not detailed here, to have a pressure sensor 15 positioned differently as long as it is able to measure said inner underpressure P. Moreover, the pressure sensor 15 may be configured to measure the inner underpressure continuously.

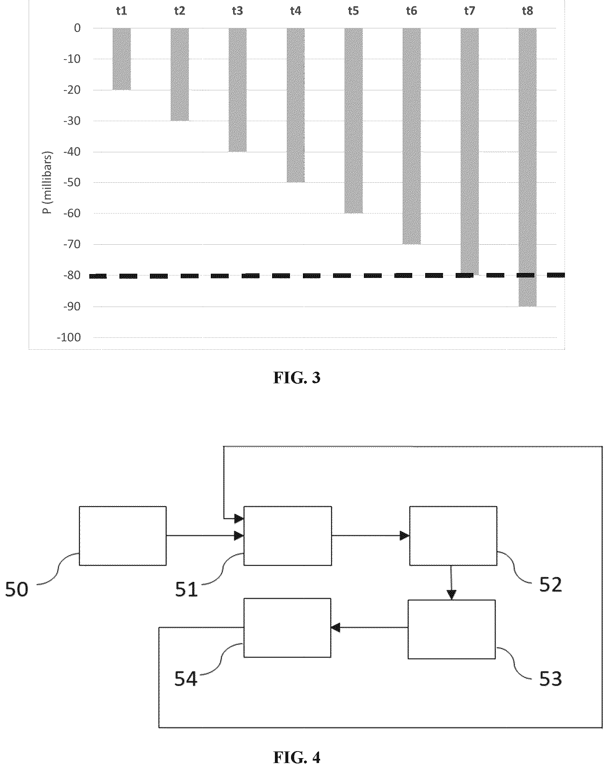

Figure 3 schematically illustrates the amplitude of the inner underpressure P (ordinate axis) as a function of time (abscissa axis) during a relaxed exhalation, for a stimulation device operated according to the prior art, i.e. without control of the delivered power.

Typically, this is a situation in which an operator (who may be the subject) has, for example, selected a predetermined power program before use, this program corresponding to a constant power operation during a relaxed exhalation.

In this example, each bar corresponds to the amplitude of the inner underpressure P when generating a negative pressure pulse, and has a width representative of the duration during which the negative pressure pulse is emitted. It should be noted that the bars are here regularly spaced, which corresponds to a configuration where negative pressure pulses are emitted at a fixed frequency, each pulse being associated with a time tᵢ of emission (in this example, index i goes from 1 to 8). As shown in Figure 3, the amplitude of the inner underpressure P increases, in absolute value, each time a negative pressure pulse is generated. This is due to the fact that the volume of air in the lungs decreases while the power delivered remains constant. Also, it is noted that the inner underpressure P differs from the underpressure shown in Figure 2 because the underpressure shown in Figure 3 corresponds to the underpressure associated to only one pulse, i.e. independently from any closed system in which such a underpressure is created.

The system of the invention aims to control an increase such as the one observed, for example, in Figure 3, by performing a method for controlling the power during each relaxed exhalation.

To this end, the system comprises, in addition to the pressure sensor 15, a plurality of modules, namely an acquisition module 31, a calculation module 32 and a control module 33.

Each module 31, 32, 33 comprises one or more processors and storage means (magnetic hard disk, electronic memory, optical disk, etc.) in which data and a computer program product are stored, in the form of a set of program code instructions to be executed to implement all or part of said control method. Alternatively, or in addition, each module 31, 32, 33 comprises one or more programmable logic circuits (FPGA, PLD, etc.), and/or one or more specialized integrated circuits (ASIC), and/or a set of discrete electronic components, etc. suitable for implementing all or part of the control method.

In other words, each module comprises a set of means configured by software (specific computer program product) and/or hardware (FPGA, PLD, ASIC, etc.) in order to implement steps of the control method which can thus be executed automatically. According to a preferred embodiment, and as illustrated in Figure 1, all the modules, i.e. the acquisition module 31, the calculation module 32 and the control module 33, are integrated into the pressure unit 13 as specific electronic circuits. Such an embodiment allows to improve the compactness of the assembly formed by the control system and the stimulation device 10.

The choice of having all the modules integrated into the pressure unit 13 is only one variation of the invention's realization. In a variant, all or part of the modules 31, 32, 33 may be positioned outside the pressure unit 13. To this end, when a module 31, 32, 33 is positioned remotely from the pressure unit 13, it includes communication means known per se (wired or wireless communication means) to communicate with the other modules, as well as with the pressure unit 13 and the pressure sensor 15.

By way of a non-limiting example (not illustrated in the figures), all modules 31, 32, 33 are positioned remotely from the pressure unit 13 and are assembled into a single unit configured to be physically connected to the stimulation device 10, for example more specifically to the pressure unit 13.

According to yet another example, both the acquisition module 31 and the control module 33 are integrated into the pressure unit 13, whereas the calculation module 32 is positioned remotely from the latter. In such a configuration, the calculation module 32 corresponds to calculation means integrated with one or more computer servers positioned near the stimulation device 10, or with one or more remote computer servers according to a cloud computing solution.

Figure 4 schematically illustrates the main steps of a method according to the technology for controlling the stimulation device 10.

In this method, each of the modules 31, 32, 33 of the system plays a particular role. As illustrated in Figure 4, the control method first comprises, during a relaxed exhalation of the subject, a generation step 50 in which a set of negative pressure pulses is generated by means of the pressure unit 13. Said control method further comprises, for at least a subset of pulses of said set, steps of:
- measuring 51, after each pulse of the subset of pulses, the inner underpressure P, associated with said pulse, by means of the pressure sensor 15,
- acquiring 52 the inner underpressure P measured during the measuring step 50, by means of the acquisition module 31,
- comparing 53 the measured inner underpressure P with a predetermined threshold S and, based on the comparison result, determining an updated power value to be applied to the pressure unit 13 so that a subsequent inner underpressure P resulting from said updated power value is, in absolute value, below the absolute value of the threshold S, said comparing and determining step 52 being performed by means of the calculation module 32,
- applying 54 the updated power value to the pressure unit 13 before the generation of the next pulse, by means of the control module 33.

As mentioned above, the steps following the generation step 50, i.e. the step 51 of measurement of the inner underpressure P, the step 52 of acquisition of the inner underpressure P, the step 53 of comparison of the measured inner underpressure P with a predetermined threshold S and determination of an updated power value, and the step 54 of application of the updated power value, are performed for at least a subset of pulses of the whole set of negative pressure pulses, and after each generated pulse of said subset. This means that after each pulsation of said subset, steps 51, 52, 53 and 54 are performed before next pulsation, *i.e.* in real-time, and then iterated.

By "at least a subset", it is referred to the fact that considering a subset grouping a plurality of pulses is equivalent to considering a plurality of subsets whose union (from an algebraic point of view) groups said plurality of pulses. For example, and for purely illustrative purposes, if the set of pulsations is denoted {P₁, P₂, P₃, P₄}, then the subset corresponding to {P₁, P₃, P₄} is considered equivalent, with regard to the implementation of the invention, to the union of the subsets corresponding to {P₁} and to {P₃, P₄} respectively.

For the rest of the description, it is considered in a non-limiting way that the threshold S is a value corresponding to a comfort limit determined during use of the stimulation device 10 by the subject. In other words, it corresponds to a tolerance threshold S of the subject with regard to the operation of the stimulation device 10. Typically, such a tolerance threshold S represents the level of underpressure beyond which, when the negative pressure pulses continue for long enough, the subject feels respiratory discomfort, forcing him/her to remove the tip 12 from the mouth in order to inhale. For example, this respiratory discomfort reflects an unpleasant sensation felt by the subject in the chest. In addition, or alternatively, the discomfort felt is related to the fact that the subject feels the need to cough. It is therefore understandable that a threshold defined in this way varies according to each subject.

According to a particular embodiment, said threshold S is a predefined value determined by the subject, alone or with the help of a physician, during a test phase of the stimulation device 10. Proceeding in this way increases the autonomy of the subject with regard to the use of the stimulation device 10, since once the test phase is over, the presence of the physician is no longer required. Moreover, when the subject uses the stimulation device 10 for the first time, the threshold S may have been set, for example, on the basis of an average value determined during a test campaign previously carried out on voluntary subjects.

In a general manner, a different type of threshold may be considered, the choice of a type of threshold constituting only a variant of implementation of the invention. For example, the threshold S may correspond to a value defined not according to the feelings of the subject, but only on the basis of a previous measurement of the maximum volume of air contained in the lungs of the subject.

In a preferred embodiment, the threshold is ranging from -15 millibars to -40 millibars, preferably -20 millibars to -40 millibars.

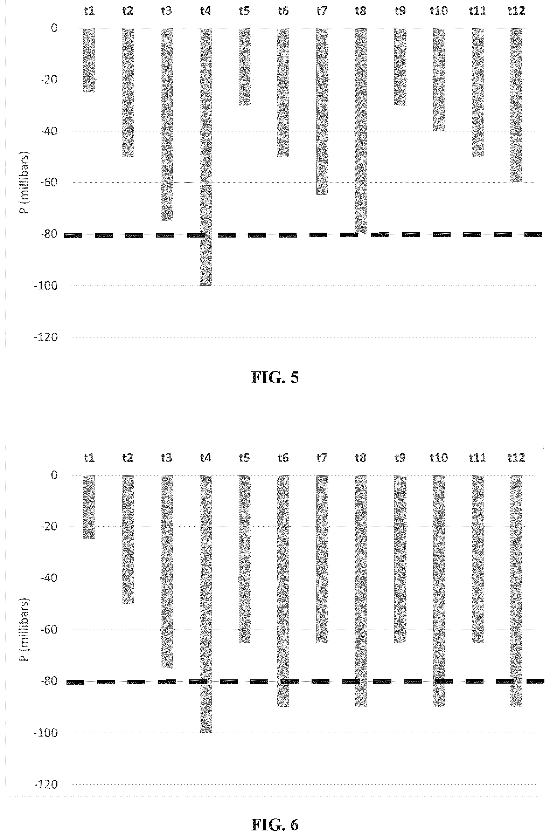

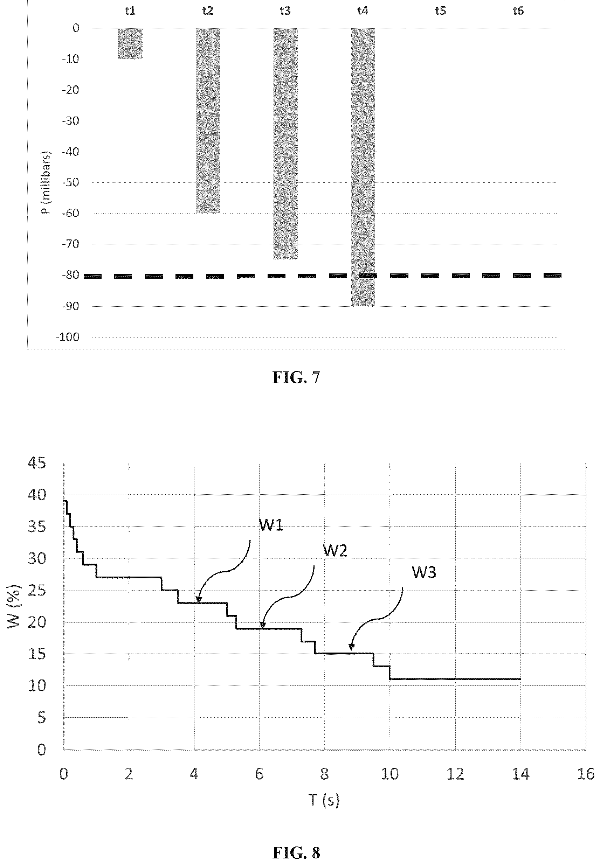

As indicated above, the control method performs its steps for at least one subset of the whole pulsation set, and possible examples of such subsets are described in more details below. In addition, embodiments of the steps illustrated in Figure 4 are described in more details below, considering: an example in which the updated power value corresponds to a decreased power value (Figure 5); an example in which the inner underpressure P oscillates around the threshold S (Figure 6); an example in which the updated power value corresponds to an increased power value (Figure 7).

### A) Particular embodiments for power decrease

According to a particular embodiment, the calculation module 32 is configured to determine, during the comparison and determination step 53, if the absolute value of the measured inner underpressure P exceeds the absolute value of the threshold S, and, in case of exceeding, determine a decreased power value to be applied to the pressure unit 13. For example, the absolute value of the threshold S is subtracted from the absolute value of the measured inner underpressure P, so that if the resulting difference is positive, an exceeding is detected. The resulting difference is referred to as the "comparison result".

In a more specific embodiment, the calculation module 32 is configured to determine the decreased power value as a function of a difference between the measured inner underpressure P and the threshold S, so that the subsequent inner underpressure P is associated to a power value of the pressure unit decreased by a value of 1% to 10% of the full range of power value of the pressure unit. For example, said difference is equal to the resulting difference calculated during the comparison 53. The choice of such a range advantageously reduces the inner underpressure P sufficiently, so that the subject does not feel any respiratory discomfort and can therefore continue to use the stimulation device 10. In other words, the subject does not feel the need to remove the tip 12 of the mouth, so that his/her lungs continue to be emptied, which enhances the extraction of mucus. In addition, targeting a particular range allows the operation of the stimulation device 10 to be finely controlled by the system.

In general, the design (software/hardware) of a power command intended to be applied to the stimulation device 10 by the control module 33, in order to achieve a particular range relative to the threshold S, can be carried out according to any method known to the person skilled in the art. Thus, the choice of a particular method of designing said power command constitutes only a variant for implementing the invention.

Figure 5 schematically illustrates the amplitude of the inner underpressure P (ordinate axis) as a function of time (abscissa axis) during a relaxed exhalation, where the power of the pressure unit 13 is reduced so as to optimize mucus extraction, which corresponds to an improvement of the embodiment illustrated in Figure 3.

As illustrated in Figure 5, twelve negative pressure pulses are emitted at a fixed frequency during a relaxed exhalation. The pulsation emission times are noted respectively t₁, t₂, ..., t₁₂. The inner underpressure P associated with each pulse is symbolized by a bar whose length is representative of the amplitude of said inner underpressure P. Below each bar is written the value of the associated amplitude. The power of the pressure unit 13 is initially set so that the amplitude of the inner underpressure P associated with a pulse is -25 millibars. The threshold S is set at -80 millibars and represented by a dotted line. The threshold value may be adjusted to -70 millibars, -60 millibars, preferably -40 millibars, or more preferably -30 millibars, depending on the subject. In addition, the calculation module 32 is configured to generate a decreased power value, further applied to the pressure unit 13 thank to the control module 33, so that when the threshold S is reached, the inner underpressure P associated with the next pulse is about -30 millibars. This decreased power value defines the power level of the device 10 for the continuation of the process, as long as no other updated power value is calculated. It can then be observed that two decreased power values are calculated during the whole relaxed exhalation, respectively at times t₄ and t₈, and that:
- the decreased power value calculated at time t₄ corresponds to a power decrease from -80 millibars to -30 millibars,
- the decreased power value calculated at time t₈ corresponds to a power decrease from -80 millibars to -30 millibars. It has to be noted that the lung of subject has been emptied during pulsations 1 to 8: this explains that the same power value of the pressure unit yields different and increasing inner underpressures.

Finally, the relaxed exhalation ends at time t₁₂ without the threshold S being reached again.

Proceeding in this way is advantageous, because if no decreased power value had been generated, the inner underpressure P would have continued to increase gradually from t₄ until the subject could no longer tolerate the discomfort caused, so that the relaxed exhalation would have ended prematurely well before t₁₂.

It should be noticed that in the example in Figure 5, it is considered that the inner underpressure P increases with increments that are not modified, unless a power reduction control is generated. It is important to note that this is only a variant for implementing the invention. In particular, all embodiments concerning the generation of a decreased power value, and described with respect to the present invention, are compatible with the embodiments relating to an increased power value, which are described in more detail below.

Moreover, in the example of Figure 5, the subset of negative pressure pulses, for which the measurement 51, acquisition 52, comparison and determination 53, and application 54 steps have been performed, consists of the pulses emitted at times t₄ and t₈ respectively. It should be noted, however, that other embodiments are possible regarding the composition of said subset, as long as they contribute to maximize the exhalation time.

For example, it is possible to have a subset with successive pulses (for example at times t₄, t₅, etc.), so that once the threshold S is exceeded a first time, it is never exceeded again during the relaxed exhalation. It is also possible to have a subset with more spaced/less spaced pulses compared to the example of Figure 5, as well as more pulses than two or even only once.

Figure 6 schematically illustrates a variant embodiment of that of Figure 5, where:
- the power of the pressure unit 13 is initially set so that the inner underpressure P associated with a pulse is -25 millibars,
- the calculation module 32 is configured to determine a decreased power value so that when the threshold S is reached, the inner underpressure P associated with the next pulse is about -65 millibars,
- next pulse is generated, with inner underpressure P about -65 millibars, below the threshold S,
- then, the power value of the pressure device is reset to the power value corresponding to inner underpressure P above the threshold S and a pulse is generated,
- the subset of last two pulses below and above threshold S is iterated.

It is therefore understood that, in this example, the power control is a local (*i.e*. temporary) control that does not decrease the power of the stimulation device 10 for the rest of the relaxed exhalation.

As shown in Figure 6, said subset comprises pulses associated respectively to t₄, t₆, t₈, t₁₀ and t₁₂. Therefore, the amplitude of the inner underpressure P oscillates around the threshold S. Proceeding in this way is particularly advantageous when the subject can tolerate the device's operation, so that it is not necessary to reduce the power too much. Oscillating around the threshold S optimizes the use of the stimulation device 10 by maximizing not only the duration of a relaxed exhalation, but also the power with which the mucus is dislodged.

It is noted that the choice of a subset consisting of pulses generated once every two represents only one variant of embodiment of the disclosure.

In a general manner, said subset may consist of pulses selected according to a particular period of oscillation around the threshold S.

The rest of the description concerns other particular embodiments for power decrease, which remain compatible with all the technical features described above with respect to the choice of the subset structure.

According to another particular embodiment, the power decrease determined during the step of comparison and determination 53 corresponds to a decreased power value determined among a set of predetermined power values. For example, said predetermined values are substantially equal to multiples of one-tenth of the maximum power of the stimulation device 10, *i.e.* 90%, 80%, 70%, 60%, 50%, 40%, 30%, 20% and 10%. Thus, it is possible to simplify the electronic architecture of the control module 33 since the decreased power values can be pre-programmed.

According to a particular embodiment, when several decreased power values are determined by the calculation module 32 during the relaxed exhalation of the subject, said values form a constant sequence over time. In other words, the power decrease rate remains the same each time the threshold S is exceeded. For example, the power decrease is set at 25%. According to this example, if the relaxed exhalation phase starts at full power of the pressure unit 13, said stimulation device 10 will operate at 75% of its maximum power after the first threshold S exceeding, then at 50% of its maximum power after the second threshold S exceeding, and so on. Proceeding in this way ensures that the subject is not rushed during a relaxed exhalation, as this latter can more easily be accommodated with the operation of the stimulation device 10. Moreover, it should be noted that such an embodiment is not limited to the choice of a fixed value for power decrease, and remains compatible with the case where said power decrease targets a specific interval with respect to the threshold S.

### B) Particular embodiments for power increase

Every feature that has been described previously in section A concerning the power decrease of the pressure unit 13 are compatible with the following description, which concerns embodiments for increasing the power of said pressure unit 13.

According to a particular embodiment, the calculation module 32 is configured to determine whether the absolute value of the measured inner underpressure P exceeds the absolute value of the threshold S, and, in case of no exceeding, compute an increased power value to be applied to the pressure unit 13. For example, the absolute value of the threshold S is subtracted from the absolute value of the measured inner underpressure P, so that if the resulting difference is negative, no exceeding is detected. The resulting difference is referred to as the "comparison result".

In a more specific embodiment, the calculation module 32 is configured to determine the increased power value as a function of a difference between the measured inner underpressure P and the threshold S, so that the subsequent inner underpressure P is associated to a power value of the pressure unit increased by a value of 1% to 10% of the full range of power value of the pressure unit. For example, said difference is equal to the resulting difference calculated during the comparison 53. The choice of such a range advantageously prevents the stimulation device 10 from operating at a too low power rate, so that not enough air would be extracted from the lungs with each pulse. In other words, proceeding in this way increases the efficiency of the device 10 by avoiding that not enough mucus is extracted during each relaxed exhalation, until the threshold S has been reached.

Figure 7 schematically illustrates the amplitude of the inner underpressure P (ordinate axis) as a function of time (abscissa axis) during part of a relaxed exhalation, where the power increase is controlled for some of the pulsations shown and where the threshold S is reached only once. It has to be noticed that Figure 7 is constructed according to similar arrangements to those used in Figure 5, except that only part of the relaxed exhalation is represented (only four emission times are illustrated).

As illustrated in Figure 7, four negative pressure pulses are emitted at a fixed frequency during a relaxed exhalation. The pulse emission times are noted respectively t₁, t₂, t₃ and t₄. The power of the pressure unit 13 is initially set so that the inner underpressure P associated with a pulse is -10 millibars. The threshold S is set at -80 millibars. In addition, the calculation module 32 is configured to determine, once the first pulse has been emitted, an increased power value. This increased power value defines the power level of the device 10 for the continuation of the process, as long as no other updated power value is determined. Therefore, as from t₂, the inner underpressure P associated with a pulsation is then -50 millibars. Thus, the inner underpressure P following the power increase, at time t₃, is -75 millibars, which is below the threshold S. The process then continues without changing the power of said pressure unit 13. Once the threshold S has been exceeded, the calculation module 32 determines, for example, a decreased power value in accordance with the embodiments described above (not illustrated in Figure 7).

It is noted that if no power increase had been generated, the threshold S would only have been reached after a later time t₅, which may be constraining if it corresponds to a too long duration compared to the beginning of the relaxed exhalation phase.

In the example of Figure 7, the subset of negative pressure pulses, for which the measurement 51, acquisition 52, comparison and determination 53, and application 54 steps have been performed, consists in the single pulse emitted at time t₂. However, other embodiments are possible regarding the composition of said subset, as long as they contribute to maximize the exhalation time.

In a general manner, all considerations relating to the composition of the subset, which have been described in Section A, apply similarly to the case of power increase. In other words, and apart from respecting to remain below the threshold for the pulse following a power increase, nothing excludes that said subset comprises a single or several pulses, consecutive or not, etc.

### C) Particular embodiments for constant inner underpressure

Combination of features that have been described previously in section A concerning the power decrease of the pressure unit and in section B concerning the power increase of the pressure unit enables a particular embodiment of disclosure.

In this embodiment, a predetermined value of inner underpressure P₀ is selected, typically in the range from -15 millibars to -40 millibars. Exact value of P₀ is determined according to the subject characteristics such as anatomy, pathology, lung volume, sensibility to cough and according to efficiency of air extraction and/or mucus removal.

The system is then run with instruction to deliver to the subject the inner underpressure P₀, by adjustment of power value of the pressure unit. The power value of pressure unit versus time may be recorded as shown in Figure 8.

In a first stage, power value is high as extraction of air is easy and volume of air in lungs is large.

Then, power value decreases until it reaches a plateau Wₚₗₐₜ or successive plateaus of decreasing power values (shown as W1, W2 and W3 on Figure 8 for instance). It has been observed that air extraction and/or mucus removal is optimal when the plateau is long, *i.e.* when the working point (P₀;Wₚₗₐₜ) is stable for a long time.

In a last stage, power value decreases again, corresponding to the end of relaxed exhalation.

Thus, an advantageous use of the device is to record power value of pressure unit versus time so as to identify optimal working point of device for a subject.

In another advantageous use, successive records - every week or month for instance - of power value of pressure unit versus time may be compared to monitor the condition of the subject. A decrease in Wₚₗₐₜ for the same predetermined P₀ denotes an improvement of lung conditions of the subject.

While various embodiments have been described and illustrated, the detailed description is not to be construed as being limited hereto. Various modifications can be made to the embodiments by those skilled in the art without departing from the scope of the disclosure as defined by the claims.

### EXAMPLES

Example 1: Comparison between a tracheobronchial-air stimulation device with automatic control system and a tracheobronchial-air stimulation device without automatic control system.

This example was carried out with the same subject during two different treatment sessions. During the first treatment session, said subject was equipped with a tracheobronchial-air stimulation device without automatic control system, whereas, during the second treatment session, said subject was equipped with a tracheobronchial-air stimulation device with automatic control system.

During the first treatment session, a set of negative pressure pulses is generated by means of a pressure unit during a relaxed exhalation of the subject.

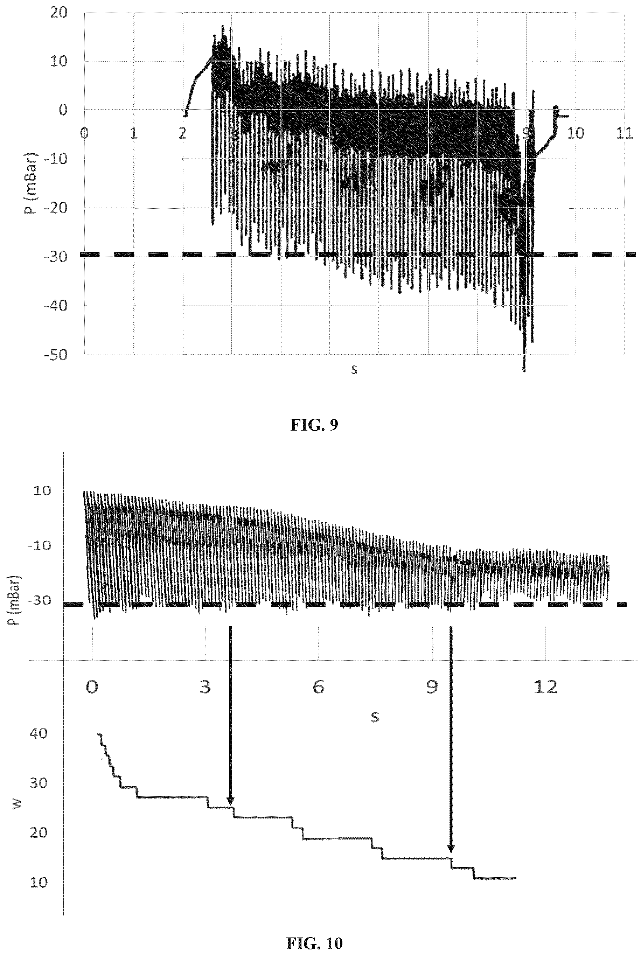

Figure 9 shows the amplitude of the inner underpressure P (ordinate axis, mBar) as a function of time (abscissa axis, seconds) during part of a relaxed exhalation, where the inner underpressure is not controlled and where the threshold S (-30 mBar, represented by a dotted line) is reached many times and exceeded.

Without an automatic control system, no correction is applied to the pressure unit when the threshold S is reached, leading to many exceeding occurrences. In this case, the inner underpressure is maintained above the threshold for too long. Due to discomfort, the relaxed exhalation is stopped prematurely by the subject because he had to cough (at 9 second on the record, corresponding to an exhalation of less than 7 second). In the end, the relaxed exhalation is not carried out for a sufficiently long period of time so that the subject completely empties the air contained in his lungs. Consequently, quantity of mucus drained during a relaxed exhalation will not be optimal, treatment is not efficient, and other treatment sessions will be needed to reach a satisfying mucus drainage. During the second treatment session, a set of negative pressure pulses is generated by means of a pressure unit during a relaxed exhalation of the subject.

According to the disclosed method (not separately claimed), during said session:
- the inner underpressure P is measured, after each pulse of a subset of pulses by means of the pressure sensor 15,
- the inner underpressure P measured during the measuring step is acquired by means of the acquisition module 31,
- the measured inner underpressure P is compared with a predetermined threshold S and, based on the comparison result, an updated power value to be applied to the pressure unit 13 is determined so that a subsequent inner underpressure P resulting from said updated power value is, in absolute value, below the absolute value of the threshold S, said comparing and determining step being performed by means of the calculation module 32,
- the updated power value is applied to the pressure unit 13 before the generation of the next pulse, by means of the control module 33.

The step of measurement of the inner underpressure P, the step of acquisition of the inner underpressure P, the step of comparison of the measured inner underpressure P with a predetermined threshold S and determination of an updated power value, and the step of application of the updated power value are performed before next pulsation, *i.e.* in real-time, and then iterated.

Figure 10 shows in upper part the amplitude of the inner underpressure P (ordinate axis) and in lower part the power value W of the pressure unit (as a percentage of full power of pressure unit), both as a function of time (abscissa axis) during part of a relaxed exhalation, where the inner underpressure is controlled.

It can be observed that, when the absolute value of the measured inner underpressure P exceeds the absolute value of the threshold S (-30 mBar, represented by a dotted line), a decreased power value is determined and applied in real-time to the pressure unit 13 (see arrows on Figure 10). This results in real-time decreasing of the inner underpressure P so that it goes back below the threshold S.

In this case, the excess of inner underpressure does not continue at least for the next pulsation, thus preventing the inner underpressure from being maintained for too long above the threshold, so that the relaxed exhalation is not stopped prematurely. The automatic control system for a tracheobronchial-air stimulation device of the invention allows the relaxed exhalation is carried out for a sufficiently long period of time (here more than 12 second) so that the subject completely empties the air contained in his lungs, which facilitates the drainage of mucus during a relaxed exhalation.

Using the automatic control system with the tracheobronchial-air stimulation device, the optimal inner underpressure is maintained constant throughout the pulses, whereas, without the control system, the maximum inner underpressure fluctuates a lot. This results in a high respiratory discomfort for the subject who may feel the need to stop the treatment session prematurely.

As shown on Figure 10, with the automatic control, the amplitude of inner underpressure P is decreased so that the amplitude of inner underpressure P at the end of the relaxed exhalation is smaller than the amplitude of inner underpressure P at the beginning of the same relaxed exhalation. This allows a smooth ending of the relaxed exhalation by preparing the lungs to return to normal pressure, and prevents the need of the subject to cough. Contrarily, it can be observed on Figure 9 that, without the automatic control, the amplitude of inner underpressure P stays about constant throughout the relaxed exhalation. This may lead to a traumatic ending for the subject compared to a relaxed exhalation performed with the assistance of the automatic control system of the invention.

## Claims

1. Automatic control system for a tracheobronchial-air stimulation device (10) intended to be connected to a subject, the stimulation device (10) comprising a pressure unit (13) configured to generate a set of negative pressure pulses during a relaxed exhalation of the subject, each pulse corresponding to a power of the pressure unit (13), and a connection assembly (11, 12) configured to connect the pressure unit (13) to the respiratory system of the subject, wherein the control system comprises:
- a pressure sensor (15) configured to measure, after each pulse generated by the pressure unit (13), an inner underpressure (P) applied to the respiratory system of the subject corresponding to said pulse,
- a calculation module (32) configured to compare in real-time the absolute value of the measured inner underpressure (P) with a predetermined threshold (S) and, based on the comparison result, determine an updated power value to be applied to the pressure unit (13) so that the absolute value of a subsequent inner underpressure (P) corresponding to the updated power value is below the absolute value of the threshold (S),
- a control module (33) configured to apply the updated power value to the pressure unit (13) before generation of the next pulse.

2. System according to claim **1,** wherein the inner pressure is the pressure at the entrance of the respiratory system of the subject.

3. System according to claim **1** or **2,** further comprising an acquisition module (31) configured to acquire, during a relaxed exhalation of the subject and after each pulse generated by the pressure unit (13), a value of the measured inner underpressure (P) associated with said pulse.

4. System according to any one of the preceding claims, wherein the calculation module (32) is configured to determine if the absolute value of the measured inner underpressure (P) exceeds the absolute value of the threshold (S), and, in case of exceeding, determine a decreased power value to be applied to the pressure unit (13).

5. System according to claim **4,** wherein the calculation module (32) is configured to determine the decreased power value as a function of a difference between the absolute value of the measured inner underpressure (P) and the absolute value of the threshold (S), so that the absolute value of a subsequent inner underpressure (P) corresponding to said decreased power value is decreased by a value of between 10 mbar et 60 mbar compared to the latter measured inner underpressure (P).

6. System according to any one of claims **4** or **5,** wherein the decreased power value is determined among a set of predetermined power values, in particular such that each predetermined power value is substantially equal to a multiple of one-tenth of the maximum power of the pressure unit (13).

7. System according to any one of the preceding claims, wherein the calculation module (32) is configured to determine whether the absolute value of the measured inner underpressure (P) exceeds the absolute value of the threshold (S), and, in case of no exceeding, determine an increased power value to be applied to the pressure unit (13).

8. System according to claim **7,** wherein the calculation module (32) is configured to determine the increased power value as a function of a difference between the absolute value of the measured inner underpressure (P) and the absolute value of the threshold (S), so that the subsequent inner underpressure (P) corresponding to said increased power value is increased by a value of between 10 mbar et 60 mbar compared to the latter measured inner underpressure (P).

9. System according to any of the preceding claims, wherein the pressure unit (13) is configured in such a way that, for a plurality of relaxed exhalations of the subject, a first set of consecutive exhalations is associated with pulses generated by the pressure unit (13) at a first frequency (f₁), whereas a second set of consecutive exhalations is associated with pulses generated by the pressure unit (13) at a second frequency (f₂) distinct from the first frequency (f₁).

10. System according to claim **9,** wherein the first frequency (f₁) is equal to 12 Hz and the second frequency (f₂) is equal to 6 Hz.

11. System according to any of the preceding claims, wherein the threshold (S) is a value corresponding to a comfort limit determined during use of the stimulation device (10) by the subject.

12. System according to any of the preceding claims, wherein the threshold (S) is a predefined value determined during a test phase of the stimulation device (10).

13. An assembly comprising:
- a tracheobronchial-air stimulation device (10) intended to be connected to a subject, the stimulation device (10) including a pressure unit (13) configured to generate, during a relaxed exhalation of the subject, a set of negative pressure pulses, each pulse corresponding to a power of the pressure unit (13), and a connection assembly (11, 12) configured to connect the pressure unit (13) to the respiratory system of the subject, and
- an automatic control system according to any one of the preceding claims.

14. Computer program comprising instructions for the implementation of at least the calculation steps of a method for automatically controlling a tracheobronchial-air stimulation device (10) as a function of measurements of an inner underpressure (P) when the program is executed by a computer, the stimulation device (10) comprising a pressure unit (13) configured to generate a set of negative pressure pulses during a relaxed exhalation of a subject, each pulse corresponding to a power of the pressure unit (13), and a connection assembly (11, 12) configured to connect the pressure unit (13) to the respiratory system of the subject, wherein the method comprises steps of:
- measuring (51), after each pulse generated by the pressure unit (13), an inner underpressure (P) applied to the respiratory system of the subject corresponding to said pulse,
- comparing (53) in real-time the absolute value of the measured inner underpressure (P) with a predetermined threshold (S) and, based on the comparison result, determining an updated power value to be applied to the pressure unit (13) so that the absolute value of a subsequent inner underpressure (P) corresponding to the updated power value is below the absolute value of the threshold (S),
- applying (54) the updated power value to the pressure unit (13) before generation of the next pulse.

15. Non-transitory computer readable medium comprising instructions for the implementation of at least the calculation steps of a method for automatically controlling a tracheobronchial-air stimulation device (10) as a function of measurements of an inner underpressure (P) when the instructions are executed by a computer, the stimulation device (10) comprising a pressure unit (13) configured to generate a set of negative pressure pulses during a relaxed exhalation of a subject, each pulse corresponding to a power of the pressure unit (13), and a connection assembly (11, 12) configured to connect the pressure unit (13) to the respiratory system of the subject, wherein the method comprises steps of:
- measuring (51), after each pulse generated by the pressure unit (13), an inner underpressure (P) applied to the respiratory system of the subject corresponding to said pulse,
- comparing (53) in real-time the absolute value of the measured inner underpressure (P) with a predetermined threshold (S) and, based on the comparison result, determining an updated power value to be applied to the pressure unit (13) so that the absolute value of a subsequent inner underpressure (P) corresponding to the updated power value is below the absolute value of the threshold (S),
- applying (54) the updated power value to the pressure unit (13) before generation of the next pulse.

## Patentansprüche

1. Automatisches Steuersystem für eine tracheobronchiale Luftstimulationsvorrichtung (10), die dazu bestimmt ist, an einen Probanden angeschlossen zu werden, wobei die Stimulationsvorrichtung (10) eine Druckeinheit (13), die so eingerichtet ist, dass sie während einer entspannten Ausatmung des Probanden eine Reihe von Unterdruckimpulsen erzeugt, wobei jeder Impuls einer Leistung der Druckeinheit (13) entspricht, und eine Verbindungsbaugruppe (11, 12) umfasst, die so eingerichtet ist, dass sie die Druckeinheit (13) mit dem Atmungssystem des Probanden verbindet, wobei das Steuersystem Folgendes umfasst:
- einen Drucksensor (15), der so eingerichtet ist, dass er nach jedem von der Druckeinheit (13) erzeugten Impuls einen inneren Unterdruck (P), der auf das Atmungssystem des Probanden angewendet wird, misst, der dem Impuls entspricht,
- ein Berechnungsmodul (32), das so eingerichtet ist, dass es in Echtzeit den absoluten Wert des gemessenen inneren Unterdrucks (P) mit einem vorbestimmten Schwellenwert (S) vergleicht und basierend auf dem Vergleichsergebnis einen aktualisierten Leistungswert bestimmt, der auf die Druckeinheit (13) anzuwenden ist, so dass der absolute Wert eines nachfolgenden inneren Unterdrucks (P), der dem aktualisierten Leistungswert entspricht, unter dem absoluten Wert des Schwellenwerts (S) liegt,
- ein Steuermodul (33), das so eingerichtet ist, dass es den aktualisierten Leistungswert vor der Erzeugung des nächsten Impulses auf die Druckeinheit (13) anwendet.

2. System nach Anspruch **1,** wobei der Innendruck der Druck am Eingang des Atemsystems des Probanden ist.

3. System nach Anspruch **1** oder **2,** ferner umfassend ein Erfassungsmodul (31), das so eingerichtet ist, dass es während einer entspannten Ausatmung des Probanden und nach jedem von der Druckeinheit (13) erzeugten Impuls einen Wert des gemessenen inneren Unterdrucks (P), der mit dem Impuls verbunden ist, erfasst.

4. System nach einem der vorhergehenden Ansprüche, wobei das Berechnungsmodul (32) so eingerichtet ist, dass es bestimmt, ob der absolute Wert des gemessenen inneren Unterdrucks (P) den absoluten Wert des Schwellenwerts (S) überschreitet, und bei Überschreitung einen verringerten Leistungswert bestimmt, der auf die Druckeinheit (13) anzuwenden ist.

5. System nach Anspruch **4,** wobei das Berechnungsmodul (32) so eingerichtet ist, dass es den verringerten Leistungswert als Funktion einer Differenz zwischen dem absoluten Wert des gemessenen inneren Unterdrucks (P) und dem absoluten Wert des Schwellenwerts (S) bestimmt, so dass der absolute Wert eines nachfolgenden inneren Unterdrucks (P), der dem verringerten Leistungswert entspricht, um einen Wert zwischen 10 mbar und 60 mbar gegenüber dem zuletzt gemessenen inneren Unterdruck (P) verringert wird.

6. System nach einem der Ansprüche **4** oder **5,** wobei der verringerte Leistungswert aus einer Reihe vorbestimmter Leistungswerte bestimmt wird, insbesondere so, dass jeder vorbestimmte Leistungswert im Wesentlichen einem Vielfachen von einem Zehntel der Maximalleistung der Druckeinheit (13) entspricht.

7. System nach einem der vorhergehenden Ansprüche, wobei das Berechnungsmodul (32) so eingerichtet ist, dass es bestimmt, ob der absolute Wert des gemessenen inneren Unterdrucks (P) den absoluten Wert des Schwellenwerts (S) überschreitet, und, falls dieser nicht überschritten wird, einen erhöhten Leistungswert bestimmt, der auf die Druckeinheit (13) anzuwenden ist.

8. System nach Anspruch **7,** wobei das Berechnungsmodul (32) so eingerichtet ist, dass es den erhöhten Leistungswert als Funktion einer Differenz zwischen dem absoluten Wert des gemessenen inneren Unterdrucks (P) und dem absoluten Wert des Schwellenwerts (S) bestimmt, so dass der nachfolgende innere Unterdruck (P), der dem erhöhten Leistungswert entspricht, um einen Wert zwischen 10 mbar und 60 mbar gegenüber dem zuletzt gemessenen inneren Unterdruck (P) erhöht wird.

9. System nach einem der vorhergehenden Ansprüche, wobei die Druckeinheit (13) so eingerichtet ist, dass für eine Vielzahl von entspannten Ausatmungen des Probanden ein erster Satz aufeinanderfolgender Ausatmungen mit Impulsen verbunden ist, die von der Druckeinheit (13) mit einer ersten Frequenz (f₁) erzeugt werden, während ein zweiter Satz aufeinanderfolgender Ausatmungen mit Impulsen verbunden ist, die von der Druckeinheit (13) mit einer zweiten Frequenz (f₂) erzeugt werden, die sich von der ersten Frequenz (f₁) unterscheidet.

10. System nach Anspruch **9,** wobei die erste Frequenz (f₁) gleich 12 Hz und die zweite Frequenz (f₂) gleich 6 Hz ist.

11. System nach einem der vorhergehenden Ansprüche, wobei der Schwellenwert (S) ein Wert ist, der einer Komfortgrenze entspricht, die während der Verwendung der Stimulationsvorrichtung (10) durch den Probanden bestimmt wird.

12. System nach einem der vorhergehenden Ansprüche, wobei der Schwellenwert (S) ein vordefinierter Wert ist, der während einer Testphase der Stimulationsvorrichtung (10) bestimmt wird.

13. Baugruppe, umfassend:
- eine tracheobronchiale Luftstimulationsvorrichtung (10), die dazu bestimmt ist, an einen Probanden angeschlossen zu werden, wobei die Stimulationsvorrichtung (10) eine Druckeinheit (13) einschließt, die so eingerichtet ist, dass sie während einer entspannten Ausatmung des Probanden eine Reihe von Unterdruckimpulsen erzeugt, wobei jeder Impuls einer Leistung der Druckeinheit (13) entspricht, und eine Verbindungsbaugruppe (11, 12), die so eingerichtet ist, dass sie die Druckeinheit (13) mit dem Atmungssystem des Probanden verbindet, und
- ein automatisches Steuersystem nach einem der vorhergehenden Ansprüche.

14. Computerprogramm, umfassend Anweisungen zur Durchführung von mindestens den Berechnungsschritte eines Verfahrens zur automatischen Steuerung einer tracheobronchialen Luftstimulationsvorrichtung (10) als Funktion von Messungen eines inneren Unterdrucks (P), wenn das Programm von einem Computer ausgeführt wird, wobei die Stimulationsvorrichtung (10) eine Druckeinheit (13) umfasst, die so eingerichtet ist, dass sie während einer entspannten Ausatmung eines Probanden eine Reihe von Unterdruckimpulsen erzeugt, wobei jeder Impuls einer Leistung der Druckeinheit (13) entspricht, und einer Verbindungsbaugruppe (11, 12), die so eingerichtet ist, dass sie die Druckeinheit (13) mit dem Atmungssystem des Probanden verbindet, wobei das Verfahren folgende Schritte umfasst:
- Messen (51), nach jedem von der Druckeinheit (13) erzeugten Impuls, eines inneren Unterdrucks (P), der auf das Atmungssystem des Probanden angewendet wird, der dem Impuls entspricht,
- Vergleichen (53) in Echtzeit des absoluten Werts des gemessenen inneren Unterdrucks (P) mit einem vorbestimmten Schwellenwert (S) und, basierend auf dem Vergleichsergebnis, Bestimmen eines aktualisierten Leistungswerts, der auf die Druckeinheit (13) anzuwenden ist, so dass der absolute Wert eines nachfolgenden inneren Unterdrucks (P), der dem aktualisierten Leistungswert entspricht, unter dem absoluten Wert des Schwellenwerts (S) liegt,
- Anwenden (54) des aktualisierten Leistungswerts auf die Druckeinheit (13) vor Erzeugung des nächsten Impulses.

15. Nicht vorübergehendes rechnerlesbares Medium, umfassend Anweisungen zur Durchführung von mindestens den Berechnungsschritten eines Verfahrens zur automatischen Steuerung einer tracheobronchialen Luftstimulationsvorrichtung (10) als Funktion von Messungen eines inneren Unterdrucks (P), wenn die Anweisungen von einem Computer ausgeführt werden, wobei die Stimulationsvorrichtung (10) eine Druckeinheit (13) umfasst, die so eingerichtet ist, dass sie während einer entspannten Ausatmung eines Probanden eine Reihe von Unterdruckimpulsen erzeugt, wobei jeder Impuls einer Leistung der Druckeinheit (13) entspricht, und einer Verbindungsbaugruppe (11, 12), die so eingerichtet ist, dass sie die Druckeinheit (13) mit dem Atmungssystem des Probanden verbindet, wobei das Verfahren folgende Schritte umfasst:
- Messen (51), nach jedem von der Druckeinheit (13) erzeugten Impuls, eines inneren Unterdrucks (P), der auf das Atmungssystem des Probanden angewendet wird, der dem Impuls entspricht,
- Vergleichen (53) in Echtzeit des absoluten Werts des gemessenen inneren Unterdrucks (P) mit einem vorbestimmten Schwellenwert (S) und, basierend auf dem Vergleichsergebnis, Bestimmen eines aktualisierten Leistungswerts, der auf die Druckeinheit (13) anzuwenden ist, so dass der absolute Wert eines nachfolgenden inneren Unterdrucks (P), der dem aktualisierten Leistungswert entspricht, unter dem absoluten Wert des Schwellenwerts (S) liegt,
- Anwenden (54) des aktualisierten Leistungswerts auf die Druckeinheit (13) vor Erzeugung des nächsten Impulses.

## Revendications

1. Système de commande automatique pour un dispositif de stimulation aérienne trachéobronchique (10) destiné à être connecté à un sujet, le dispositif de stimulation (10) comprenant une unité de pression (13) configurée pour générer un ensemble d'impulsions de pression négative pendant une expiration relâchée du sujet, chaque impulsion correspondant à une puissance de l'unité de pression (13), et un ensemble de connexion (11, 12) configuré pour connecter l'unité de pression (13) au système respiratoire du sujet, le système de commande comprenant :
- un capteur de pression (15) configuré pour mesurer, après chaque impulsion générée par l'unité de pression (13), une dépression interne (P) appliquée au système respiratoire du sujet correspondant à ladite impulsion,
- un module de calcul (32) configuré pour comparer en temps réel la valeur absolue de la dépression interne mesurée (P) à un seuil prédéterminé (S) et, en fonction du résultat de la comparaison, déterminer une valeur de puissance actualisée à appliquer à l'unité de pression (13) de sorte que la valeur absolue d'une dépression interne ultérieure (P) correspondant à la valeur de puissance actualisée soit inférieure à la valeur absolue du seuil (S),
- un module de commande (33) configuré pour appliquer la valeur de puissance actualisée à l'unité de pression (13) avant la génération de l'impulsion suivante.

2. Système selon la revendication 1, dans lequel la pression interne est la pression à l'entrée du système respiratoire du sujet.

3. Système selon la revendication 1 ou 2, comprenant en outre un module d'acquisition (31) configuré pour acquérir, lors d'une expiration relâchée du sujet et après chaque impulsion générée par l'unité de pression (13), une valeur de la dépression interne mesurée (P) associée à ladite impulsion.

4. Système selon l'une quelconque des revendications précédentes, dans lequel le module de calcul (32) est configuré pour déterminer si la valeur absolue de la dépression interne mesurée (P) dépasse la valeur absolue du seuil (S), et, en cas de dépassement, déterminer une valeur de puissance diminuée à appliquer à l'unité de pression (13).

5. Système selon la revendication 4, dans lequel le module de calcul (32) est configuré pour déterminer la valeur de puissance diminuée en fonction d'une différence entre la valeur absolue de la dépression interne mesurée (P) et la valeur absolue du seuil (S), de sorte que la valeur absolue d'une dépression interne ultérieure (P) correspondant à ladite valeur de puissance diminuée soit diminuée d'une valeur comprise entre 10 mbar et 60 mbar par rapport à cette dernière dépression interne mesurée (P).

6. Système selon l'une quelconque des revendications 4 ou 5, dans lequel la valeur de puissance diminuée est déterminée parmi un ensemble de valeurs de puissance prédéterminées, notamment de telle sorte que chaque valeur de puissance prédéterminée soit sensiblement égale à un multiple d'un dixième de la puissance maximale de l'unité de pression (13).

7. Système selon l'une quelconque des revendications précédentes, dans lequel le module de calcul (32) est configuré pour déterminer si la valeur absolue de la dépression interne mesurée (P) dépasse la valeur absolue du seuil (S), et, en cas de non-dépassement, déterminer une valeur de puissance augmentée à appliquer à l'unité de pression (13).

8. Système selon la revendication 7, dans lequel le module de calcul (32) est configuré pour déterminer la valeur de puissance augmentée en fonction d'une différence entre la valeur absolue de la dépression interne mesurée (P) et la valeur absolue du seuil (S), de sorte que la dépression interne ultérieure (P) correspondant à ladite valeur de puissance augmentée soit augmentée d'une valeur comprise entre 10 mbar et 60 mbar par rapport à cette dernière dépression interne mesurée (P).

9. Système selon l'une quelconque des revendications précédentes, dans lequel l'unité de pression (13) est configurée de telle sorte que, pour une pluralité d'expirations relâchées du sujet, un premier ensemble d'expirations consécutives est associé à des impulsions générées par l'unité de pression (13) à une première fréquence (f₁), tandis qu'un deuxième ensemble d'expirations consécutives est associé à des impulsions générées par l'unité de pression (13) à une deuxième fréquence (f₂) distincte de la première fréquence (f₁).

10. Système selon la revendication 9, dans lequel la première fréquence (f₁) est égale à 12 Hz et la deuxième fréquence (f₂) est égale à 6 Hz.

11. Système selon l'une quelconque des revendications précédentes, dans lequel le seuil (S) est une valeur correspondant à une limite de confort déterminée lors de l'utilisation du dispositif de stimulation (10) par le sujet.

12. Système selon l'une quelconque des revendications précédentes, dans lequel le seuil (S) est une valeur prédéfinie déterminée lors d'une phase de test du dispositif de stimulation (10).

13. Un ensemble comprenant:
- un dispositif de stimulation aérienne trachéobronchique (10) destiné à être connecté à un sujet, le dispositif de stimulation (10) comprenant une unité de pression (13) configurée pour générer, lors d'une expiration relâchée du sujet, un ensemble d'impulsions de pression négative, chaque impulsion correspondant à une puissance de l'unité de pression (13), et un ensemble de connexion (11, 12) configuré pour connecter l'unité de pression (13) au système respiratoire du sujet, et
- un système de commande automatique selon l'une quelconque des revendications précédentes.

14. Programme d'ordinateur comprenant des instructions pour la mise en oeuvre d'au moins les étapes de calcul d'une méthode de commande automatique pour un dispositif de stimulation aérienne trachéobronchique (10) comme une fonction des mesures d'une dépression interne (P) lorsque le programme est executé par un ordinateur, le dispositif de stimulation (10) comprenant une unité de pression (13) configurée pour générer un ensemble d'impulsions de pression négative pendant une expiration relâchée du sujet, chaque impulsion correspondant à une puissance de l'unité de pression (13), et un ensemble de connexion (11, 12) configuré pour connecter l'unité de pression (13) au système respiratoire du sujet,, dans lequel la methode comprend les étapes de:
- mesurer (51), après chaque impulsion générée par l'unité de pression (13), une dépression interne (P) appliquée au système respiratoire du sujet correspondant à ladite impulsion,
- comparer (53) en temps réel la valeur absolue de la dépression interne mesurée (P) à un seuil prédéterminé (S) et, en fonction du résultat de la comparaison, déterminer une valeur de puissance actualisée à appliquer à l'unité de pression (13) de sorte que la valeur absolue d'une dépression interne ultérieure (P) correspondant à la valeur de puissance actualisée soit inférieure à la valeur absolue du seuil (S),
- appliquer (54) la valeur de puissance actualisée à l'unité de pression (13) avant la génération de l'impulsion suivante.

15. Support non-transitoire lisible par ordinateur comprenant des instructions pour la mise en oeuvre d'au moins les étapes de calcul d'une méthode de commande automatique pour un dispositif de stimulation aérienne trachéobronchique (10) comme une fonction des mesures d'une dépression interne (P) lorsque les instructions sont executées par un ordinateur, le dispositif de stimulation (10) comprenant une unité de pression (13) configurée pour générer un ensemble d'impulsions de pression négative pendant une expiration relâchée du sujet, chaque impulsion correspondant à une puissance de l'unité de pression (13), et un ensemble de connexion (11, 12) configuré pour connecter l'unité de pression (13) au système respiratoire du sujet,, dans lequel la methode comprend les étapes de:
- mesurer (51), après chaque impulsion générée par l'unité de pression (13), une dépression interne (P) appliquée au système respiratoire du sujet correspondant à ladite impulsion,
- comparer (53) en temps réel la valeur absolue de la dépression interne mesurée (P) à un seuil prédéterminé (S) et, en fonction du résultat de la comparaison, déterminer une valeur de puissance actualisée à appliquer à l'unité de pression (13) de sorte que la valeur absolue d'une dépression interne ultérieure (P) correspondant à la valeur de puissance actualisée soit inférieure à la valeur absolue du seuil (S),
- appliquer (54) la valeur de puissance actualisée à l'unité de pression (13) avant la génération de l'impulsion suivante.
